(19) **Europäisches Patentamt European Patent Office Office européen des brevets**

(11) **EP 3 095 450 B1**

(12) **EUROPEAN PATENT SPECIFICATION**

(45) Date of publication and mention of the grant of the patent:
**23.10.2019 Bulletin 2019/43**

(51) Int Cl.:
**A61K 35/28** *(2015.01)*      **A61P 11/00** *(2006.01)*
**A61P 17/02** *(2006.01)*

(21) Application number: **16171424.1**

(22) Date of filing: **21.03.2008**

(54) **MESENCHYMAL STEM CELLS FOR USE IN IMPROVING PULMONARY FUNCTION**

MESENCHYMALE STAMMZELLEN ZUR VERWENDUNG IN DER VERBESSERUNG DER LUNGENFUNKTION

CELLULES SOUCHES MÉSENCHYMATEUSES POUR UTILISATION DANS L'AMELIORATION DE LA FONCTION PULMONAIRE

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **22.03.2007 US 726676**

(43) Date of publication of application:
**23.11.2016 Bulletin 2016/47**

(62) Document number(s) of the earlier application(s) in accordance with Art. 76 EPC:
**09009947.4 / 2 123 747**
**08744188.7 / 2 051 718**

(73) Proprietor: **Mesoblast International Sàrl**
**1217 Meyrin (CH)**

(72) Inventors:
• **Aggarwal, Sudeepta**
**North Potomac MD Maryland 20878 (US)**
• **Pittenger, Mark F.**
**Severna Park MD Maryland 21146 (US)**
• **Varney, Timothy**
**Baltimore MD Maryland 21229 (US)**
• **Danilkovitch, Alla**
**Columbia MD Maryland 21045 (US)**

(74) Representative: **Schnappauf, Georg**
**ZSP Patentanwälte PartG mbB**
**Hansastraße 32**
**80686 München (DE)**

(56) References cited:
**WO-A-00/06701          WO-A-2005/093044**

**WO-A1-2007/124594**

• **DATABASE WPI Week 200679 Thomson Scientific, London, GB; AN 2006-781676 XP002545400, & WO 2006/112365 A (JAPAN HEALTH SCI FOUND) 26 October 2006 (2006-10-26)**
• **ROJAS M ET AL: "Bone Marrow-Derived Mesenchymal Stem Cells in Repair of the Injured Lung", AMERICAN JOURNAL OF RESPIRATORY CELL AND MOLECULAR BIOLOGY, AMERICAN LUNG ASSOCIATION, NEW YORK, NY, US, vol. 33, no. 2, 1 August 2005 (2005-08-01), pages 145-152, XP003015826, ISSN: 1044-1549**
• **ORTIZ L A ET AL: "Mesenchymal stem cell engraftment in lung is enhanced bleomycin exposure and ameliorates its fibrotic effects", PROCEEDINGS OF THE NATIONAL ACADEMY OF SCIENCES OF USA, NATIONAL ACADEMY OF SCIENCE, WASHINGTON, DC, US, vol. 100, no. 14, 8 July 2003 (2003-07-08), pages 8407-8411, XP003015827, ISSN: 0027-8424**
• **WANG GUOSHUN ET AL: "Adult stem cells from bone marrow stroma differentiate into airway epithelial cells: Potential therapy for cystic fibrosis", PROCEEDINGS OF THE NATIONAL ACADEMY OF SCIENCES OF THE UNITED STATES OF AMERICA, vol. 102, no. 1, 4 January 2005 (2005-01-04), pages 186-191, XP002545399, ISSN: 0027-8424**
• **CONESE M ET AL: "Stem cells and cystic fibrosis", JOURNAL OF CYSTIC FIBROSIS, ELSEVIER, vol. 5, no. 3, 1 August 2006 (2006-08-01), pages 141-143, XP024978019, ISSN: 1569-1993 [retrieved on 2006-08-01]**

EP 3 095 450 B1

**Description**

BACKGROUND OF THE INVENTION

This invention relates to mesenchymal stem cells for use in treating a disease selected from chronic obstructive pulmonary disease (COPD) and acute respiratory distress syndrome (ARDS) in an animal.

Mesenchymal stem cells (MSCs) are multipotent stem cells that can differentiate readily into lineages including osteoblasts, myocytes, chondrocytes, and adipocytes (Pittenger, et al., Science, vol. 284, pg. 143 (1999); Haynesworth, et al., Bone, vol. 13, pg. 69 (1992); Prockop, Science, vol. 276, pg. 71 (1997)). *In vitro* studies have demonstrated the capability of MSCs to differentiate into muscle (Wakitani, et al., Muscle Nerve, vol. 18,, pg. 1417 (1995)), neuronal-like precursors (Woodbury, et al., J. Neurosci. Res., vol. 69, pg. 908 (2002); Sanchez-Ramos, et al., Exp. Neurol., vol. 171, pg. 109 (2001)), cardiomyocytes (Toma, et al., Circulation, vol. 105, pg. 93 (2002); Fakuda, Artif. Organs, vol. 25, pg. 187 (2001)) and possibly other cell types. In addition, MSCs have been shown to provide effective feeder layers for expansion of hematopoietic and embryonic stem cells (Eaves, et al., Ann, N.Y. Acad. Sci., vol. 938, pg. 63 (2001); Wagers, et al., Gene Therapy, vol. 9, pg. 606 (2002)). Recent studies with a variety of animal models have shown that MSCs may be useful in the repair or regeneration of damaged bone, cartilage, meniscus or myocardial tissues (DeKok, et al., Clin. Oral Implants Res., vol. 14, pg. 481 (2003)); Wu, et al., Transplantation, vol. 75, pg. 679 (2003); Noel, et al., Curr. Opin. Investig. Drugs, vol. 3, pg. 1000 (2002); Ballas, et al., J. Cell. Biochem. Suppl., vol. 38, pg. 20 (2002); Mackenzie, et al., Blood Cells Mol. Dis., vol. 27 (2002)). Several investigators have used MSCs with encouraging results for transplantation in animal disease models including osteogenesis imperfecta (Pereira, et al., Proc. Nat. Acad. Sci., vol. 95, pg. 1142 (1998)), parkinsonism (Schwartz, et al., Hum. Gene Ther., vol. 10, pg. 2539 (1999)), spinal cord injury (Chopp, et al., Neuroreport, vol. 11, pg. 3001 (2000); Wu, et al., J. Neurosci. Res., vol. 72, pg. 393 (2003)) and cardiac disorders (Tomita, et al., Circulation, vol. 100, pg. 247 (1999). Shake, et al., Ann. Thorac. Sure., vol. 73, pg. 1919 (2002)). Importantly, promising results also have been reported in clinical trials for osteogenesis imperfecta (Horwitz, et al., Blood, vol, 97, pg. 1227 (2001); Horowitz, et al. Proc. Nat. Acad. Sci., vol. 99, pg. 8932 (2002)) and enhanced engraftment of heterologous bone marrow transplants (Frassoni, et al., Int. Society for Cell Therapy, SA006 (abstract) (2002); Koc, et al., J. Clin. Oncol., vol. 18, pg. 307 (2000)).

MSCs express major histocompatibility complex (MHC) class I antigen on their surface but do not express MHC class II (Le Blanc, et al., Exp. Hematol., vol. 31, pg. 890 (2003); Potian, et al., J. Immunol., vol. 171, pg. 3426 (2003)) and no B7 or CD40 co-stimulatory molecules (Majumdar, et al., J. Biomed. Sci., vol. 10, pg. 228 (2003)), suggesting that these cells have a low-immunogenic phenotype (Tse, et al., Transplantation, vol. 75, pg. 389 (2003)). MSCs also inhibit 1-cell proliferative responses in an MHC-independent manner (Bartholomew, et al., Exp. Hematol., vol. 30, pg. 42 (2002); Devine, et al., Cancer J., vol. 7, pg. 576 (2001); DiNicola, et al., Blood, vol. 99, pg, 3838 (2002)). These immunological properties of MSCs may enhance their transplant engraftment and limit the ability of the recipient immune system to recognize and reject allogeneic cells following transplantation. The production of factors by MSCs, that modulate the immune response and support hematopoiesis together with their ability to differentiate into appropriate cell types under local stimuli make them desirable stem cells for cellular transplantation studies (Majumdar, et al., Hematother. Stem Cell Res., vol. 9, pg. 841 (2000); Haynesworth, et al., J. Cell. Physiol., vol. 166, pg. 585 (1996).

BRIEF SUMMARY OF THE INVENTION

Applicants presently have examined the interactions of mesenchymal stem cells with isolated immune cell populations, including dendritic cells (DC1 and DC2), effector T-cells (Th1 and Th2), and NK cells. Based on such interactions, Applicants discovered that mesenchymal stem cells may regulate the production of various factors that may regulate several steps in the immune response process. Thus, the mesenchymal stem cells may be employed in the treatment of disease conditions and disorders involving the immune system, or diseases, conditions, or disorders involving inflammation, epithelial damage, or allergic responses. Such diseases, conditions, and disorders are selected from COPD and ARDS.

In addition, it is believed that mesenchymal stem cells express and secrete vascular endothelial growth factor, or VEGF, which promotes angiogenesis by stimulating the formation of new blood vessels. Mesenchymal stem cells also stimulate peripheral blood mononuclear cells (PBMCs) to produce VEGF.

Furthermore, it is believed that mesenchymal stem cells stimulate dendritic cells (DCs) to produce Interferon-Beta (IFN-$\beta$), which promotes tumor suppression and immunity against viral infection.

DETAILED DESCRIPTION OF THE INVENTION

In one embodiment, the animal to which the mesenchymal stem cells are administered is a mammal. The mammal may be a primate, including human and non-human primates.

In general, the mesenchymal stem cell (MSC) therapy is based, for example, on the following sequence: harvest of MSC-containing tissue, isolation and expansion of MSCs, and administration of the MSCs to the animal, with or without biochemical or genetic manipulation.

The mesenchymal stem cells that are administered may be a homogeneous composition or may be a mixed cell population enriched in MSCs. Homogeneous mesenchymal stem cell compositions may be obtained by culturing adherent marrow or periosteal cells, and the mesenchymal stem cell compositions may be obtained by culturing adherent marrow or periosteal cells, and the mesenchymal stem cells may be identified by specific cell surface markers which are identified with unique monoclonal antibodies. A method of obtaining a cell population enriched in mesenchymal stem cells is described, for example, in U.S. Patent No. 5,486,359. Alternative sources for mesenchymal stem cells include, but are not limited to, blood, skin, cord blood, muscle, fat, bone, and perichondrium.

The mesenchymal stem cells may be administered by a variety of procedures. The mesenchymal stem cells may be administered systemically, such as by intravenous, intraarterial, or intraperitoneal administration.

The mesenchymal stem cells may be from a spectrum of sources including autologous, allogeneic, or xenogeneic.

The mesenchymal stem cells may be administered in conjunction with an acceptable pharmaceutical carrier. For example, the mesenchymal stem cells may be administered as a cell suspension in a pharmaceutically acceptable liquid medium or gel for injection or topical application.

The mesenchymal stem cells are administered to an animal to treat inflammation which results from a lung disease or disorder selected from Acute Respiratory Distress Syndrome (ARDS) and Chronic Obstructive Pulmonary Disease (COPD).

Although the scope of this embodiment is not to be limited to any theoretical reasoning, the inflammatory response in the above-mentioned lung diseases or disorders involves the secretion of TNF-alpha and/or MCP-1. It is believed that the mesenchymal stem cells migrate to inflamed lung tissue due to increased production of TNF-alpha and/or MCP-1, which are chemoattractants for mesenchymal stem cells.

It is to be understood, however, that the scope of this aspect of the present invention is not to be limited to the treatment of any particular inflammatory response.

The mesenchymal stem cells are administered in an amount effective to treat an inflammatory response in an animal. The mesenchymal stem cells may be administered in an amount of from about $1 \times 10^5$ cells/kg to about $1 \times 10^7$ cells/kg. In another embodiment, the mesenchymal stem cells are administered in an amount of from about $1 \times 10^6$ cells/kg to about $5 \times 10^6$ cells/kg. The exact dosage of mesenchymal stem cells to be administered is dependent upon a variety of factors, including the age, weight, and sex of the patient, the inflammatory response being treated, and the extent and severity thereof.

The mesenchymal stem cells may be administered in conjunction with an acceptable pharmaceutical carrier, as hereinabove described.

In accordance with the present invention, there is provided a method of treating lung disorders or diseases having fibrotic and may include in addition, inflammatory components, selected from, Acute Respiratory Distress Syndrome (ARDS) and Chronic Obstructive Pulmonary Disease (COPD).

The mesenchymal stem cells, in one embodiment, are administered to the animal in order to improve pulmonary function due to pulmonary diseases or diseases in other organs leading to pulmonary insufficiency or lung fibrosis. Such diseases have fibrotic, and may also have in addition, inflammatory and/or immunological components, and are selected from Acute Respiratory Distress Syndrome (ARDS) and Chronic Obstructive Pulmonary Disease (COPD).

Acute Respiratory Distress Syndrome (ARDS) is a life threatening lung disease having a variety of causes, including but not limited to ventilator injury and sudden blunt trauma to the chest. The disease is characterized by inflammation of the lung parenchyma, resulting in impaired gas exchange and concomitant expression and secretion of inflammatory mediators. These inflammatory mediators include TNF-alpha, IL-1, IL-8, and monocyte chemoattractant protein-1 or MCP-1. TNF-alpha and MCP-1 are chemoattractants for mesenchymal stem cells. Thus, increased expression of TNF-alpha and MCP-1 in the damaged lung will facilitate mesenchymal stem cell recruitment to the area of lung tissue damage.

Chronic Obstructive Pulmonary Disease, or COPD, is a major cause of illness and death worldwide. COPD is characterized by airflow obstruction due to chronic bronchitis or emphysema. COPD is characterized by a thickening of the alveolar walls and inflammation, resulting in a loss of elasticity in and damage to the alveolar tissue, as well as clogging of the lung bronchi with mucus deposits.

The inflammatory response in COPD includes the local secretion of IL-6, IL-1 Beta, TNF-alpha, and MCP-1. Although the scope of the present invention is not intended to be limited to any theoretical reasoning, it is believed that the mesenchymal stem cells migrate to the damaged lung tissue in COPD patients due to increased production in the damaged lung of the chemoattractants TNF-alpha and MCP-1.

In addition, increased neutrophil infiltration is characteristic of COPD, and neutrophilic inflammation is resistant to current COPD treatments, such as corticosteroid therapy. Although the scope of the present invention is not to be limited to any theoretical reasoning, it is believed that treatment with mesenchymal stem cells inhibits neutrophilic inflammation by downregulation of factors that act as chemoattractants for neutrophils.

In addition, current evidence suggests that the inflammatory component of COPD may extend to other tissues in the body. (Heaney, et al., Current Med. Chem. vol. 14, No. 7, pgs. 787-796 (2007)). Although the scope of the present invention is not intended to be limited to any theoretical reasoning, it is believed that mesenchymal stem cells home specifically to such sites as well, and participate in the local healing process.

Apoptotic death of lung cells is another result of COPD (Calabrese, et al., Respir. Res., vol. 6, pg. 14 (2005)). Mesenchymal stem cells secrete a variety of growth factors including hepatocyte growth factor (HFG) and fibroblast growth factors (FGFs), which have been shown to be beneficial for treatment of pulmonary emphysema (Shigemura, et al., Circulation, vol. 111, pg. 1407 (2005); Morino, et al., Chest, vol. 128, pg. 920 (2005)).

Although the scope of the present invention is not to be limited to any theoretical reasoning, it is believed that TNF-alpha and MCP-1 expression results in the recruitment of mesenchymal stem cells to damaged lung tissue.

Although the scope of the present invention is not intended to be limited to any theoretical reasoning, it is believed that the mesenchymal stern cells improve pulmonary function in an animal having lung fibrosis by inhibiting inflammatory responses by downregulating pro-inflammatory cytokine and chemokine secretion, resulting in a subsequent decrease in recruitment of inflammatory cells to the site. It also is believed that the mesenchymal stem cells inhibit the immune response in those lung disorders which elicit an immune response, thereby preventing cell-mediated as well as soluble factor mediated tissue cell killing.

The mesenchymal stem cells also facilitate tissue repair by protection of tissue cells from apoptosis, and stimulate cell proliferation and mobilization of tissue-specific stem cells via secretion of growth factors such as HGF, VEGF, and FGFs. In addition, the mesenchymal stem cells prevent pathological remodeling and scar formation in the lung tissue.

More particularly, it is believed that the mesenchymal stem cells reduce local expression of TNF-alpha, which in turn leads to a reduction in TGF-beta expression and a reduction in a recruitment of fibroblasts, which are the major cells contributing to scar formation. In addition, the mesenchymal stem cells remodel the existing lung scar tissue and/or prevent expansion of the scar though the expression and local secretion of matrix metalloproteinases (MMPs). The enzymatic activity of MMPs leads to degradation of extracellular matrix proteins, including those proteins that are included in scar tissue.

The mesenchymal stem cells may be genetically engineered with one or more polynucleotides encoding a therapeutic agent. The polynucleotides may be delivered to the mesenchymal stem cells via an appropriate expression vehicle. Expression vehicles which may be employed to genetically engineer the mesenchymal stem cells include, but are not limited to, retroviral vectors, adenoviral vectors, and adeno-associated virus vectors.

The selection of an appropriate polynucleotide encoding a therapeutic agent is dependent upon various factors, including the disease or disorder being treated, and the extent and severity thereof. Polynucleotides encoding therapeutic agents, and appropriate expression vehicles are described further in U.S. Patent No. 6,355,239.

It is to be understood that the mesenchymal stem cells, when employed in the above-mentioned therapies and treatments, may be employed in combination with other therapeutic agents known to those skilled in the art, including, but not limited to, growth factors, cytokines, drugs such as anti-inflammatory drugs, and cells other than mesenchymal stem cells, such as dendritic cells, and may be administered with soluble carriers for cells such as hyaluronic acid, or in combination with solid matrices, such collagen, gelatin, or other biocompatible polymers, as appropriate.

It is to be understood that the methods described herein may be carried out in a number of ways and with various modifications and permutations thereof that are well known in the art. It also may be appreciated that any theories set forth as to modes of action or interactions between cell types should not be construed as limiting this invention in any manner, but are presented such that the methods of the invention can be understood more fully.

BRIEF DESCRIPTION OF SEVERAL VIEWS OF THE DRAWINGS

The invention now will be described with respect to the drawings, wherein:

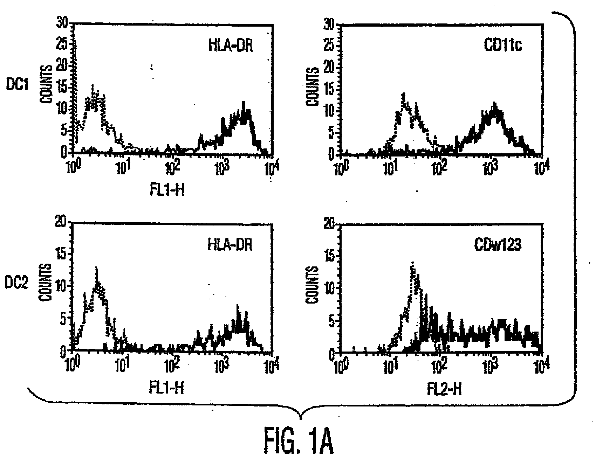

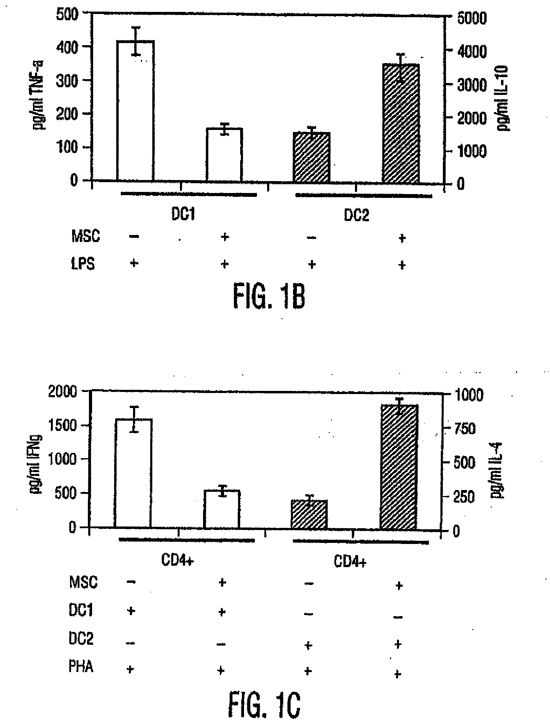

Fig. 1 MSCs modulate dendritic cell functions. (A) Flow cytometric analysis of mature monocytic DC1 cells using antibodies against HLA-DR and CD11c and of plasmacytoid DC2 cells using antibodies against HLA-DR and CD123 (IL-3 receptor). (---): isotype control; (-): FITC/PE conjugated antibodies. (B) MSCs inhibit TNF-$\alpha$ secretion (primary y-axis) and increase 1L-10 secretion (secondary y-axis) from activated DC1 and DC2 respectively. (C) MSCs cultured with mature DC1 cells inhibit IFN-$\gamma$ secretion (primary y-axis) by T cells and increase IL-4 levels (secondary y-axis) as compared to MSC or DC alone. The decreased production of pro-inflammatory IFN-$\gamma$ and increased production of anti-inflammatory IL-4 in the presence of MSCs indicated a shift in the T cell population towards an anti-inflammatory phenotype.

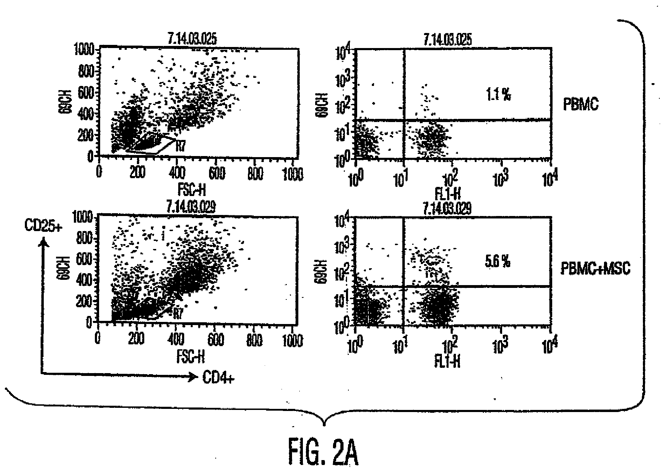

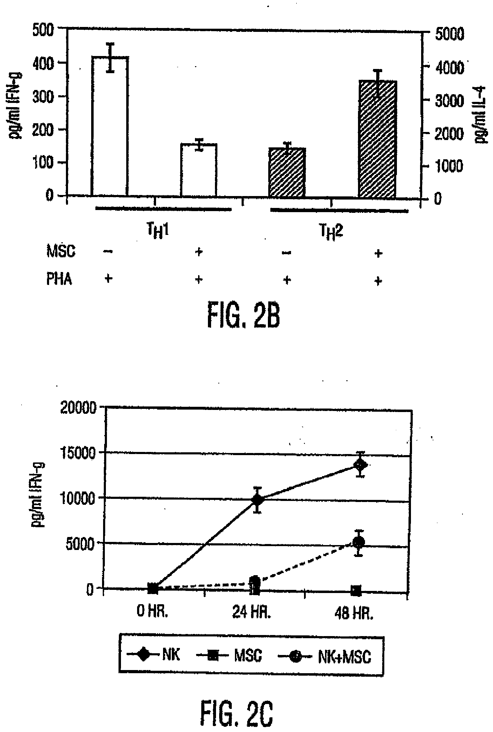

Fig. 2 MSCs inhibit pro-inflammatory effector T cell function. (A) Flow cytometric analysis of $T_{Reg}$ cell numbers (in %) by staining PBMCs or non-adherent fraction in MSC+PBMC culture (MSC+PBMC) with FITC-conjugated CD4 (x-axis) and PE conjugated CD25 (y-axis) antibodies. Gates were set based on isotype control antibodies as background. Graphs are representative of 5 independent experiments. (B) $T_H1$ cells generated in presence of MSCs secreted reduced levels of IFN-$\gamma$ (primary Y-axis) and $T_H2$ cells generated in presence of MSCs secreted increased amounts of IL-4 (secondary y-axis) in cell culture supernatants. (C) MSCs inhibit IFN-$\gamma$ secretion from purified NK cells cultured for 0, 24, or 48 hours in a 24-well plate. Data shown are mean$\pm$SD cytokine secretion in one experiment and are representative of 3 independent experiments.

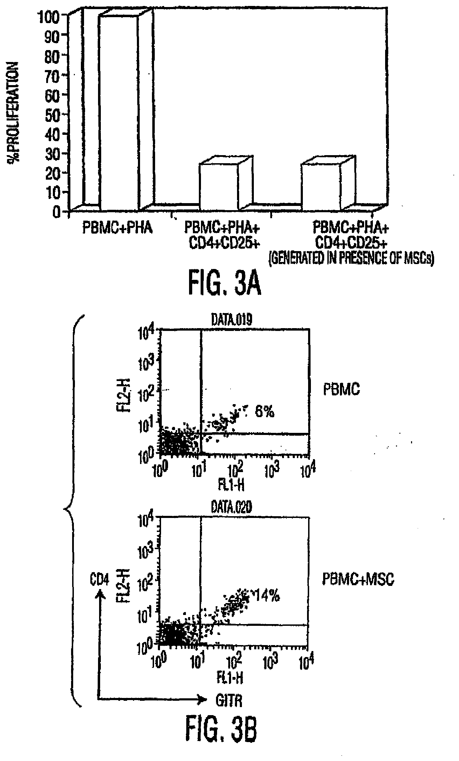

Fig. 3 MSCs lead to increased numbers of $T_{Reg}$ cell population and increased GITR expression. (A) A CD4+ CD25+ $T_{Reg}$ cell population from PBMC or MSC + PBMC (MSC to PBMC ratio 1:10) cultures (cultured without any further stimulation for 3 days) was isolated using a 2-step magnetic isolation procedure, These cells were irradiated (to block any further proliferation) and used as stimulators in a mixed lymphocyte reaction (MLR), where responders were allogeneic PBMCs (stimulator to responder ratio 1:100) in the presence of phytohemagglutinin (PHA) (2.5 mg/ml). The cells were cultured for 48 hours, following which $^3$H thymidine was added, and incorporated radioactivity was counted after 24 hours. The results showed that the $T_{Reg}$ population, generated in the presence of MSCs (lane 3) was similar functionally to the $T_{Reg}$ cells generated in the absence of MSCs (lane 2). (B) PBMCs were cultured for 3 days in the absence (top plot) or presence (bottom plot) of MSCs (MSC to PBMC ratio 1:10), following which the non-adherent fraction was harvested and immunostained with FITC-labeled GITR and PE-labeled CD4. Results show a greater than twofold increase in GITR expression in cells cultured in the presence of MSCs.

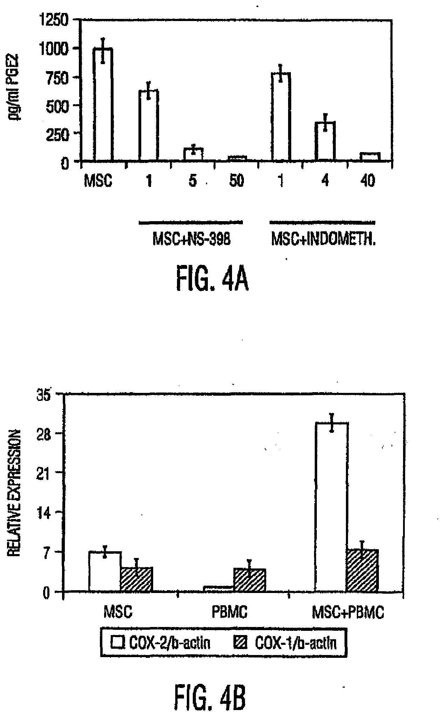

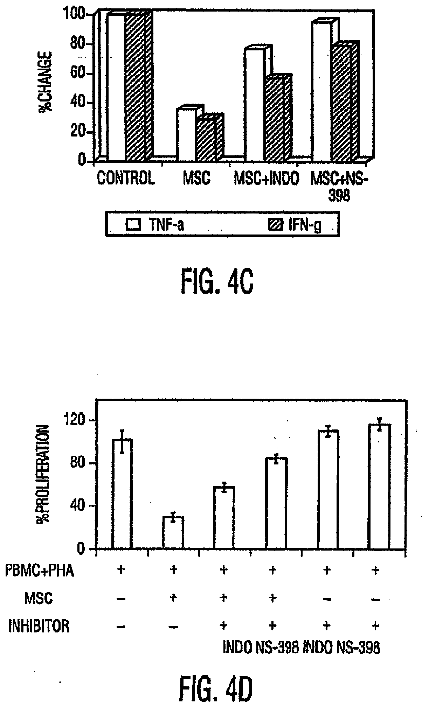

Fig. 4 MSCs produce $PGE_2$ and blocking $PGE_2$ reverses MSC-mediated immuno-modulatory effects . (A) $PGE_2$ secretion (mean$\pm$SD) in culture supernatants obtained from MSCs cultured in the presence or absence of $PGE_2$ blockers NS-398 or indomethacin (Indometh.) at various concentrations. Inhibitor concentrations are in $\mu$M and data presented are values obtained after 24 hour culture (B) COX-1 and COX-2 expression in MSCs and PBMCs using real-time RT-PCR. MSCs expressed significantly higher levels of COX-2 as compared to PBMCs, and when MSCs were cultured in presence of PBMCs, there was a >3-fold increase in COX-2 expression in MSCs. Representative data from 1 of 3 independent experiments is shown. The MSC+PBMC cultures were setup in a trans-well chamber plate where MSCs were plated onto the bottom chamber and PBMCs onto the top chamber. (C) Presence of $PGE_2$ blockers indomethacin (Ind.) or NS-398 increases TNF-$\alpha$ secretion from activated DCs ($\square$) and IFN-$\gamma$ secretion from $T_H1$ cells ($\square$) as compared to controls. Data were calculated as % change from cultures generated in absence of MSCs and $PGE_2$ inhibitors (D) Presence of $PGE_2$ blockers indomethacin (Indo) and NS-398 during MSC-PBMC co-culture (1:10) reverses MSC-mediated anti-proliferative effects on PHA-treated PBMCs. Data shown are from one experiment and are representative of 3 independent experiments.

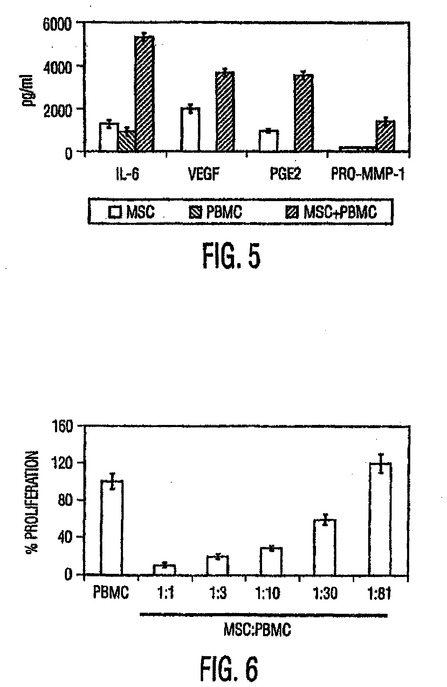

Fig. 5 Constitutive MSC cytokine secretion is elevated in the presence of allogeneic PBMCs. Using previously characterized human MSCs, the levels of the cytokines 1L-6 and VEGF, lipid mediator $PGE_2$, and matrix metalloproteinase 1 (pro MMP-1) in culture supernatant of MSCs cultured for 24 hours in the presence (hatched bars) or absence (open bars) of PBMCs (MSC to PBMC ratio 1:10) were analyzed. The MSCs produced 1L-6, VEGF, and $PGE_2$ constitutively, and the levels of these factors increased upon co-culture with PBMCs, thereby suggesting that MSCs may play a role in modulating immune functions in an inflammatory setting.

Fig. 6 MSCs inhibit mitogen-induced T-cell proliferation in a dose-dependent manner. Increasing numbers of allogeneic PBMCs were incubated with constant numbers of MSCs (2,000 cells/well) plated on a 96-well plate in the presence or absence of PHA (2.5 mg/ml) for 72 hours, and $^3$H thymidine incorporation determined (in counts per minute, or cpm). There was a dose-dependent inhibition of the proliferation of PHA-treated PBMCs in the presence of MSCs. Representative results from 1 of 3 independent experiments are shown. Similar results were reported by LeBlanc, et al., Scand J. Immunol., vol. 57, pg. 11 (2003).

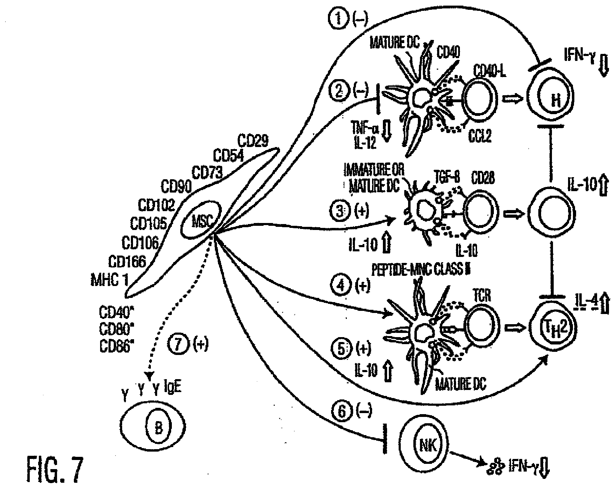

Fig. 7 Schematic diagram of proposed MSC mechanism of action, MSCs mediate their immuno-modulatory effects by affecting cells from both the innate (DCs-pathways 2-4; and NK- pathway 6) and adaptive (T- pathways 1 and 5 and B-pathway 7) immune systems. In response to an invading pathogen, immature DCs migrate to the site of potential entry, mature and acquire an ability to prime naive T cells (by means of antigen specific and co-stimulatory signals) to become protective effector T cells (cell-mediated $T_H1$ or humoral $T_H2$ immunity). During MSC-DC interaction, MSCs, by means of direct cell-cell contact or via secreted factor, may alter the outcome of immune response by limiting the ability of DCs to mount a cell-mediated response (pathway 2) or by promoting the ability to mount a humoral response (pathway 4), Also, when mature effector T cells are present, MSCs may interact with them to skew the balance of $T_H1$ (pathway 1) responses towards $T_H2$ responses (pathway 5), and probably towards an increased IgE producing B cell activity (pathway 7), desirable outcomes for suppression of GvHD and autoimmune disease symptoms. MSCs in their ability to result in an increased generation of $T_{Reg}$ population (pathway 3) may result in a tolerant phenotype and may aid a recipient host by dampening bystander inflammation in their local micro-environment. Dashed line (----) represents proposed mechanism.

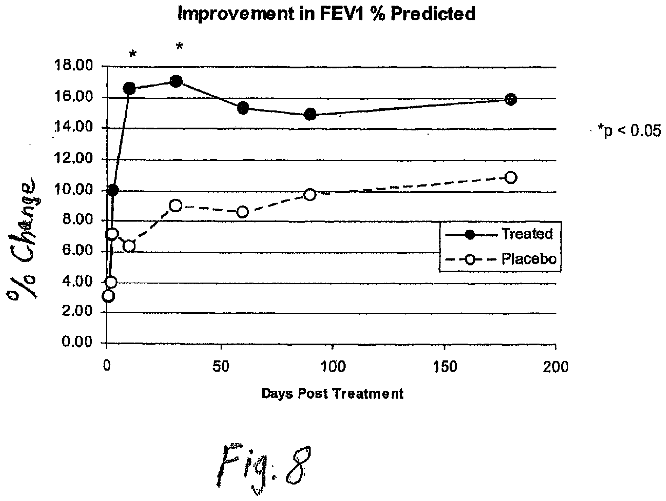

Fig. 8. MSC treatment provides an improvement in percent of predicted forced expiratory volume in one second (Pred. FEV1%) in patients treated with mesenchymal stem cells as compared to patients who received a placebo.

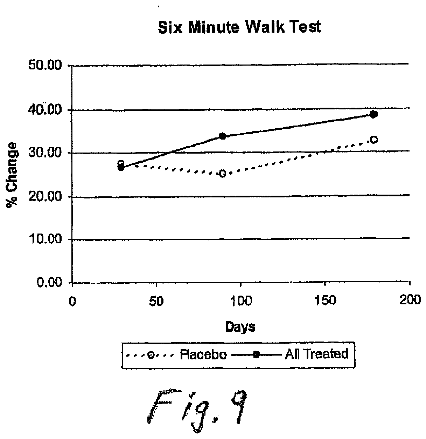

Fig. 9. Measurement of distance walked on a treadmill after six minutes. Patients who were treated with MSCs showed an increase in distance walked as compared to those who received a placebo.

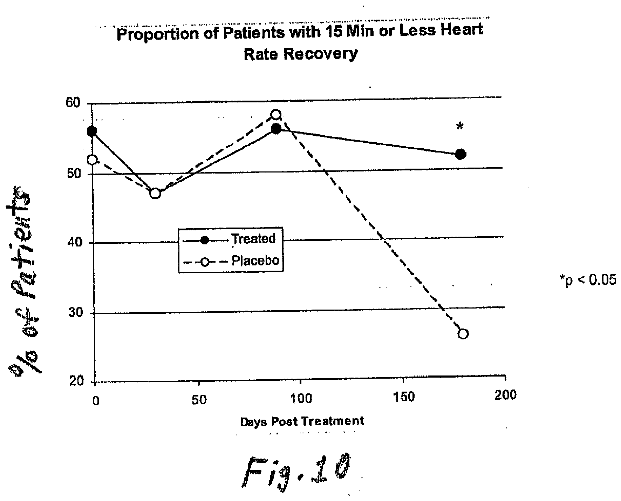

Fig. 10. Heart Rate Recovery in Patients Subjected to Treadmill Test. A greater percentage of the patients subjected to the treadmill test showed heart rate recovery to baseline values in 15 minutes or less than those who were treated with a placebo.

Examples

The invention now will be described with respect to the following examples; it is to be understood, however, that the scope of the present invention is not to be limited thereby.

Example 1

Materials and Methods Culture of human MSCs

Human MSCs were cultured as described by Pittenger et al., Science, vol, 284, pg. 143 (1999). Briefly, marrow samples were collected from the iliac crest of anonymous donors following informed consent by Poietics Technologies, Div of Cambrex Biosciences. MSCs were cultured in complete Dulbecco's Modified Eagle's Medium-Low Glucose (Life Technologies, Carlsbad, California) containing 1% antibiotic¬antimyotic solution (Invitrogen, Carlsbad, California) and 10% fetal bovine serum (FBS, JRH BioSciences, Lenexa, Kansas). MSCs grew as an adherent monolayer and were detached with trypsin/EDTA (0.05% trypsin at 37°C for 3 minutes), All MSCs used were previously characterized for multilineage potential and retained the capacity to differentiate into mesenchymal lineages (chondrocytic, adipogenic, and osteogenic) (Pittenger, et al., Science, vol. 284, pg. 143 (1999)).

**Isolation of Dendritic cells**

Peripheral blood mononuclear cells (PBMCs) were obtained from Poietics Technologies, Div of Cambrex Biosciences (Walkersville, MD). Precursors of dendritic cells (DCs) of monocytic lineage (CD1c+) were positively selected from PBMCs using a 2-step magnetic separation method according to Dzionek, et. al., J. Immunol., vol. 165, pg. 6037 (2000). Briefly, CD1c expressing B cells were magnetically depleted of CD19+ cells using magnetic beads, followed by labeling the B-cell depleted fraction with biotin-labeled CD1c (BDCA1+) and anti-biotin antibodies and separating them from the unlabeled cell fraction utilizing magnetic columns according to the manufacturer's instructions (Miltenyi Biotech, Auburn, California). Precursors of DCs of plasmacytoid lineage were isolated from PBMCs by immuno-magnetic sorting of positively labeled antibody coated cells (BDCA2+) (Miltenyi Biotech, Auburn, California).

**MSC-DC culture**

In most experiments, human MSCs and DCs were cultured in equal numbers for various time periods and cell culture supernatant collected and stored at -80°C until further evaluation. In selected experiments, MSCs were cultured with mature DC1 or DC2 cells (1:1 MSC:DC ratio) for 3 days, and then the combined cultures (MSCs and DCs) were irradiated to prevent any proliferation. Next, antibody purified, naive, allogeneic T cells (CD4+,CD45RA+) were added to the irradiated MSCs/DCs and cultured for an additional 6 days. The non-adherent cell fraction (purified T cells) was then collected from the cultures, washed twice and re-stimulated with PHA for another 24 hours, following which cell culture supernatants were harvested and analyzed for secreted IFN-$\gamma$ and IL-4 by ELISA.

**Isolation of NK cells**

Purified populations of NK cells were obtained by depleting non-NK cells that are magnetically labeled with a cocktail of biotin-conjugated monoclonal antibodies (anti - CD3, - CD14, -CD19, -CD36 and anti-IgE antibodies) as a primary reagent and anti-biotin monoclonal antibodies conjugated to Microbeads as secondary labeling reagent. The magnetically labeled non-NK cells were retained in MACS (Miltenyi Biotech, Auburn, California) columns in a magnetic field, while NK cells passed through and were collected.

**Isolation of $T_{Reg}$ cell population**

The $T_{Reg}$ cell population was isolated using a 2-step isolation procedure. First non-CD4+ T cells were indirectly magnetically labeled with a cocktail of biotin labeled antibodies and anti-biotin microbeads. The labeled cells were then depleted by separation over a MACS column (Miltenyi Biotech, Auburn, California). Next, CD4+CD25+ cells were directly labeled with CD25 microbeads and isolated by positive selection from the pre-enriched CD4+. T cell fraction. The magnetically labeled CD4+CD25+ T cells were retained on the column and eluted after removal of the column from the magnetic field.

In order to determine whether the increased CD4+CD25+ population generated in the presence of MSCs were suppressive in nature, CD4+CD25+ $T_{Reg}$ cell populations were isolated from PBMC or MSC+PBMC (MSC to PBMC ratio 1:10) cultures (cultured without any further stimulation for 3 days) using a 2-step magnetic isolation procedure. These cells were irradiated to block any further proliferation and used as stimulators in a mixed lymphocyte reaction (MLR), where responders were allogeneic PBMCs (stimulator to responder ratio 1:100) in the presence of PHA (2.5 $\mu$g/ml). The culture was carried out for 48 hours, following which $^3$H thymidine was added. Incorporated radioactivity was counted after 24 hours.

PBMCs were cultured in the absence or presence of MSCs (MSC to PBMC ratio 1:10), following which the non-adherent fraction was harvested and immunostained with FITC-labeled glucocorticoid-induced TNF receptor, or GITR, and PE -labeled CD4.

**Generation of $T_H1$/ $T_H2$ cells**

Peripheral blood mononuclear cells (PBMCs) were plated at $2\times10^6$ cells/ml for 45 min. at 37°C in order to remove monocytes. Non-adherent fraction was incubated in the presence of plate-bound anti-CD3 (5 $\mu$g/ml) and anti-CD28 (1 $\mu$g/ml) antibodies under $T_H1$ (IL-2 (4 ng/ml) IL-12 (5 ng/ml) + anti-IL-4 (1 $\mu$g/ml)) or $T_H2$ (IL-2 (4 ng/ml) + IL-4 (4 ng/ml) + anti-IFN-y (1 $\mu$g/ml)) conditions for 3 days in the presence or absence of MSCs. The cells were washed and then re-stimulated with PHA (2.5 $\mu$g/ml) for another 24 or 48 hours, following which levels of IFN-$\gamma$ and IL-4 were measured in culture supernatants by ELISA (R&D Systems, Minneapolis, Minnesota).

**Analysis of levels of VEGF, $PGE_2$, and pro-MMP-1 in culture supernatant of MSCs.**

Using previously characterized human MSCs, the levels of Interleukin-6 (IL-6), VEGF, lipid mediator prostaglandin $E_2$ ($PGE_2$), and matrix metalloproteinase 1 (pro-MMP-1) were analyzed in culture supernatant of MSCs cultured for 24 hours in the presence or absence of PBMCs (MSC to PBMC ratio 1:10).

**Proliferation of PBMCs**

Purified PBMCs were prepared by centrifuging leukopack (Cambrex, Walkersville, Maryland) on Ficoll-Hypaque (Lymphoprep, Oslo, Norway). Separated cells were cultured (in triplicates) in the presence or absence of MSCs (plated 3-4 hours prior to PBMC addition to allow them to settle) for 48 hours in presence of the mitogen PHA (Sigma Chemicals, St. Louis, Missouri). In selected experiments, PBMCs were resuspended in medium containing $PGE_2$ inhibitors Indomethacin (Sigma Chemicals, St. Louis, Missouri) or NS-938 (Cayman Chemicals, Ann Arbor, Michigan). ($^3$H)-thymidine was added (20 $\mu$l in a 200 $\mu$l culture) and the cells harvested after an additional 24 hour culture using an automatic harvester. The effects of MSCs or $PGE_2$ blockers were calculated as the percentage of the control response (100%) in presence of PHA.

**Quantitative RT-PCR**

Total RNA from cell pellets were prepared using a commercially available kit (Qiagen, Valencia, California) and according to the manufacturer's instructions. Contaminating genomic DNA was removed using the DNA-free kit (Ambion, Austin, Texas). Quantitative RT-PCR was performed on a MJ Research Opticon detection system (South San Francisco, California) using QuantiTect SYBR Green RT-PCR kit (Qiagen, Valencia, California) with primers at concentration of 0.5 $\mu$M, Relative changes in expression levels in cells cultured under different conditions were calculated by the difference in Ct values (crossing point) using $\beta$-actin as internal control. The sequence for COX-1 and COX-2 specific primers were: COX-1: 5'-CCG GAT GCC AGT CAG GAT GAT G-3'(forward), 5'-CTA GAC AGC CAG ATG CTG ACA G-3' (reverse);

7

COX-2: 5'- ATC TAC CCT CCT CAA GTC CC-3'(forward), 5'-TAC CAG AAG GGC AGG ATA CAG-3' (reverse).

Increasing numbers of allogeneic PBMCs were incubated with constant numbers of MSCs (2,000 cells/well) plated on a 96-well plate in the presence of PHA (2.5 $\mu$g/ml) for 72 hours, and $^3$H thymidine incorporation (counts per minute, cpm) was determined. The PBMCs and MSCs were cultured at ratios of MSC:PBMC of 1:1, 1:3, 1:10, 1:30, and 1:81.

**Results**

In the present studies, the interaction of human MSCs with isolated immune cell populations, including dendritic cells (DC1 and DC2), effector T cells ($T_H1$ and $T_H2$) and NK cells was examined, The interaction of MSCs with each immune cell type had specific consequences, suggesting that MSCs may modulate several steps in the immune response process. The production of secreted factor(s) that modulate and may be responsible for MSC immuno-modulatory effects was evaluated and prostaglandin synthesis was implicated.

Myeloid (DC1) and plasmacytoid (DC2) precursor dendritic cells were isolated by immuno-magnetic sorting of BDCA1$^+$ and BDCA2$^+$ cells respectively and matured by incubation with GM-CSF and IL-4 (1x10$^3$ IU/ml and 1x10$^3$ IU/ml, respectively) for DC1 cells, or IL-3 (10 ng/ml) for DC2 cells. Using flow cytometry, DC1 cells were HLA-DR$^+$ and CD11c$^+$, whereas DC2 cells were HLA-DR$^+$ and CD123$^+$ (Fig. 1A). In the presence of the inflammatory agent bacterial lipopolysaccharide (LPS, 1 ng/ml), DC1 cells produced moderate levels of TNF-$\alpha$ but when MSCs were present (ratios examined 1:1 and 1:10), there was >50% reduction in TNF-$\alpha$ secretion (Fig. 1B). On the other hand, DC2 cells produced IL-10 in the presence of LPS and its levels were increased greater than 2-fold upon MSC:DC2 co-culture (1:1) (Fig. 1B). Therefore, the MSCs modified the cytokine profile of activated DCs in culture towards a more tolerogenic phenotype. Additionally, activated DCs, when cultured with MSCs, were able to reduce IFN-$\gamma$ and increase IL-4 levels secreted by naïve CD4$^+$ T cells (Fig. 1C) suggesting a MSC-mediated shift from pro-inflammatory to anti-inflammatory T cell phenotype.

As increased IL-10 secretion plays a role in generation of regulatory cells (Kingsley, et at., J. Immunol., vol. 168, pg. 1080 (2002)), T-regulatory cells ($T_{Reg}$) were quantified by flow cytometry in co-cultures of PBMCs and MSCs. Upon culture of PBMCs with MSCs for 3-5 days, there was an increase in $T_{Reg}$ cell numbers as determined by staining of PBMCs with anti-CD4 and anti-CD25 antibodies (Fig. 2A), further supporting a MSC-induced tolerogenic response. The CD4$^+$CD25$^+$ $T_{Reg}$ cell population, generated in presence of MSCs expressed increased levels of gluocorticoid-induced TNF receptor (GITR), a cell surface receptor expressed on $T_{Reg}$ cell populations, and was suppressive in nature as it suppressed allogeneic T cell proliferation (Fig. 3A,B). Next, MSCs were investigated as to their direct ability to affect T cell differentiation. Using antibody selected purified T cells (CD4$^+$ Th cells), IFN-$\gamma$ producing $T_H1$ and IL-4 producing $T_H2$ cells were generated in presence or absence of MSCs. When MSCs were present during differentiation, there was reduced IFN-$\gamma$ secretion by $T_H1$ cells and increased IL-4 secretion by $T_H2$ cells (Fig. 2B). No significant change in IFN-$\gamma$ or IL-4 levels were seen when MSCs were added to the culture after Th cells had differentiated (at 3 days) into effector $T_H1$ or $T_H2$ types (data not shown), These experiments suggest that MSCs can affect effector T cell differentiation directly and alter the T cell cytokine secretion towards a humoral phenotype,

Similarly, when MSCs were cultured with purified NK cells (CD3-, CD14-, CD19-, CD36-) at a ratio 1:1 for different time periods (0-48 hrs), there was decreased IFN-$\gamma$ secretion in the culture supernatant (Fig. 2C), thereby suggesting that MSCs can modulate NK cell functions also.

Previous work has indicated that MSCs modify T-cell functions by soluble factor(s) (LeBlanc, et al., Exp. Hematol., vol. 31, pg. 890 (2003); Tse, et al., Transplantation, vol. 75, pg. 389 (2003). It was observed that the MSCs secreted several factors, including IL-6, prostaglandin E$_2$, VEGF and proMMP-1 constitutively, and the levels of each increased upon culture with PBMCs (Fig. 5). In order to investigate MSC-derived factors leading to inhibition of TNF-$\alpha$ and increase of IL-10 production by DCs, the potential role of prostaglandin E$_2$ was investigated, as it has been shown to inhibit TNF-$\alpha$ production by activated DCs (Vassiliou, et al., Cell. Immunol., vol. 223, pg. 120 (2003)). Conditioned media from MSC culture (24 hour culture of 0.5 x10$^6$ cells/ml) contained approx. 1000 pg/ml of PGE$_2$ (Fig. 4A). There was no detectable presence of known inducers of PGE$_2$ secretion, e.g., TNF-$\alpha$, IFN-$\gamma$ or IL-1$\beta$ (data not shown) in the culture supernatant indicating a constitutive secretion of PGE$_2$ by MSCs. The PGE$_2$ secretion by hMSCs was inhibited 60-90% in the presence of known inhibitors of PGE$_2$ production, NS-398 (5 $\mu$M) and indomethacin (4 $\mu$M) (Fig. 4A). As the release of PGE$_2$ secretion occurs as a result of enzymatic activity of constitutively active cycloxygenase enzyme 1 (COX-1) and inducible cycloxygenase enzyme 2 (COX-2) (Harris, et al., Trends Immunol., vol. 23, pg. 144 (2002)) the mRNA expression for COX-1 and COX-2 in MSCs and PBMCs using trans-well culture system was analyzed. MSCs expressed significantly higher levels of COX-2 as compared to PBMCs and the expression levels increase >3-fold upon co-culture of MSCs and PBMCs (MSC to PBMC ratio 1:10) for 24 hours (Fig. 4B). Modest changes in COX-1 levels were seen suggesting that the increase in PGE$_2$ secretion upon MSC-PBMC co-culture (Fig. 5) is mediated by COX-2 up-regulation. To investigate whether the immunomodulatory effects of MSC on DCs and T-cells were mediated by PGE$_2$) MSCs were cultured with activated dendritic cells (DC1) or $T_H1$ cells in the presence of PGE$_2$ inhibitors NS-398 or indomethacin. The presence of NS-398 or indomethacin increased TNF-$\alpha$ secretion by DCIs, and IFN-$\gamma$ secretion

from $T_H1$ cells (Fig. 4C), respectively, suggesting that MSC effects on immune cell types may be mediated by secreted $PGE_2$. Recent studies have shown that MSCs inhibit T-cell proliferation induced by various stimuli (DeNicola, et al., Blood, vol. 99, pg. 3838 (2002); LeBlanc, et al., Scand. J. Immunol., vol. 57, pg. 11 (2003)). It was observed that MSCs inhibit mitogen-induced T cell proliferation in a dose-dependent manner (Fig. 6) and when $PGE_2$ inhibitors NS-398 (5 $\mu$M) or indomethacin (4 $\mu$M) were present, there was a >70% increase in ($^3$H) thymidine incorporation by PHA-treated PBMCs in MSC containing cultures as compared to controls without inhibitors (Fig. 4D).

In summary, a model of MSC interaction with other immune cell types (Fig. 7) is proposed. When mature T cells are present, MSCs may interact with them directly and inhibit the pro-inflammatory IFN-$\gamma$ production (pathway 1) and promote regulatory T cell phenotype (pathway 3) and anti-inflammatory $T_H2$ cells (pathway 5). Further, MSCs can alter the outcome of the T cell immune response through DCs by secreting $PGE_2$, inhibiting pro-inflammatory DC1 cells (pathway 2) and promoting anti-inflammatory DC2 cells (pathway 4) or regulatory DCs (pathway 3). A shift towards $T_H2$ immunity in turn, suggests a change in B cell activity towards increased generation of IgE/IgG1 subtype antibodies (pathway 7). MSCs, by their ability to inhibit IFN-$\gamma$ secretion from NK cells likely modify NK cell function (pathway 6). This model of MSC:Immune cell interactions is consistent with the experimentation performed in several other laboratories (LeBlanc, et al., Exp. Hematol., vol. 31, pg. 890 (2003); Tse, et al., Transplantation, vol. 75, pg. 389 (2003); DiNicola, et al., Blood, vol. 99, pg. 3838 (2002)). Further examination of the proposed mechanisms is underway and animal studies are now necessary to examine the in vivo effects of MSC administration.

Reference Example 2

Mesenchymal stem cells were given to a 33-year-old female patient suffering from severe Grade IV gastrointestinal graft-versus-bost disease (GVHD). The patient was refractory to all other GVHD treatments. Endoscopic views of the patient's colon showed areas of ulceration and inflammation prior to treatment. Histology of the patient's colon showed that the graft-versus-host disease had destroyed the vast majority of the patient's intestinal crypts, prior to treatment.

The patient was given an intravenous infusion of allogeneic mesenchymal stem cells in 50 ml of Plasma Lyte A (Baxter) in an amount of 3 X $10^6$ cells per kilogram of body weight.

The patient was evaluated at two weeks post-infusion. At two weeks post-infusion, an endoscopic view of the patient's colon showed that the areas of inflammation and ulceration visible prior to treatment were resolved. In addition, a biopsy of the patient's colon showed significant regeneration of intestinal crypts. Thus, the administration of the mesenchymal stem cells to the patient resulted in a significant reduction in the inflammatory component of gastrointestinal graft-versus-host disease, and resulted in the regeneration of new functional intestinal tissue.

Example 3

9 patients received 0.5 x $10^6$ mesenchymal stem cells per kilogram of body weight, 10 patients received 1.6 x $10^6$ mesenchymal stem cells per kilogram of body weight, and 15 patients received 5.0 x $10^6$ mesenchymal stem cells per kilogram of body weight by intravenous infusion of a suspension of mesenchymal stem cells in Plasma Lyte A (Baxter) in which the mesenchymal stem cells were present in the suspension at a concentration of 2.5 x $10^6$ cells/ml. The total volume of mesenchymal stem cell suspension given thus was dependent upon the dosage of cells and the weight of the patient.

19 patients received placebo doses of Plasma Lyte A. The placebo doses were in low, medium, and high doses in proportion to the volumes of the mesenchymal stem cell suspension given to the patients treated with the mesenchymal stem cells. Of the placebo patients, 5 patients were given a low dose of the suspension, 4 patients were given a medium dose of the suspension, and 10 patients were given a high dose of the suspension.

The FEV1 spirometry test was performed over a six month period to detect potential changes related to the treatment. The testing was done according to American Thoracic Society guidelines. (Miller, et al., Eur. Respir. J., vol. 26, pgs, 319-338 (2005)).

Forced expiratory volume, or FEV1, is the maximal volume of air exhaled in the first second of a forced expiration from a position of full inspiration, expressed in liters, at body temperature (37°C), ambient pressure, saturated with water vapor (BTPS). The predicted FEV1 values were calculated for each patient based on age, sex, height, and race. The predicted FEV1 for men was calculated as follows (Crapo, et al., Am. Rev. Respir. Dis., vol. 123, pgs. 659-664 (1981):

$$\text{predicted FEV1} = 0.0414 \text{ x height (cm)} - 0.0244 \text{ x age (years)} - 2.190$$

The predicted FEV1 for women was calculated as follows (Crapo, 1981):

$$\text{predicted FEV1} = 0.0342 \times \text{height (cm)} - 0.0255 \times \text{age (years)} - 1.578$$

The above values were calculated for men and women of other than African-American background. For men and women of African-American background, the above values were multiplied by a correction factor of 0.88.

The weight of each patient also was taken. Although weight is not factored into the determination of the FEV1 values, obesity may lower the measured lung volumes, and changes in body weight can result in small changes in lung function.

FEV1 values for all patients were measured using a spirometer, which was connected to a mouthpiece or tube which was inserted into the patient's mouth. The height and weight of each patient was measured, a nose clip was placed on each patient. Each patient was instructed to inhale completely and rapidly, with a pause of less than one second at total lung capacity, and to exhale maximally until no more air can be expelled. The procedure is repeated in triplicate, and the FEV1 values for each patient were measured. From the FEV1 values, the percent of predicted FEV1 (Pred. FEV1%) values were calculated. The percent of predicted FEV1 (Pred. FEV1%) values for each patient were calculated as follows:

$$\text{Pred. FEV1\%} = 100.0 \times \frac{\text{Observed FEV1}}{\text{Predicted FEV1}}$$

The percent improvement in Pred. FEV1% values for the patients also was calculated at various time intervals up until 6 months (180 days) after treatment. The results for the MSC and placebo-treated groups are shown in Figure 8. Such results show the average percent change in Pred. FEV1 % values for all MSC-treated patients and the average percent change in Pred. FEV1% values for all patients who received the placebo.

Compared to the control group of patients, the patients who were treated with the MSCs showed a greater improvement in Pred. FEV1%, relative to baseline (pre-treatment) values, from three days through six months post-infusion. At both 10 and 30 days post-infusion, the difference in improvement in Pred. FEV1 % values observed for MSC-treated and placebo patients was significant statistically ($p < 0.05$).

MSC-treated and placebo patients also were subjected to a treadmill test in which the patients walked on a treadmill in which the distance walked by each patient was measured in six minute intervals. Distance measurements were taken after treatment (baseline), and at one month, three months, and six months post-treatment. The test was performed according to ATS (American Thoracic Society) guidelines (Am. J. Respir. Crit. Care Med., vol. 166, pg. 111 (2002)).

The average percent changes in distance walked for all MSC-treated patients and all placebo-treated patients as compared to the baseline distance are shown in Figure 9. At both the three month and six month timepoints, the MSC-treated patients showed an increase in the distance walked compared to the patients who received the placebo.

Following the six minute treadmill test that was given to the patients shortly after treatment, and at one, three, and six months after treatment, heart rate recovery for the patients was determined.

The heart rate recovery results are shown in Figure 10. As shown in Figure 10, at six months after the treatment, the difference in the percentage of patients who received the MSC treatments showing heart rate recovery to baseline values within 15 minutes after the cessation of the treadmill walking as compared to the patients who received the placebo treatments was significant statistically.

The above results with respect to improvement in Pred. FEV1%, distance walked, and heart rate recovery, show that the patients treated with the mesenchymal stem cells had improved pulmonary functions as compared to the placebo group. The above results suggest that fibrotic lung diseases or disorders may be treated with mesenchymal stem cells, whereby the mesenchymal stem cells improve pulmonary function, reduce existing scar tissue in the lung, and/or prevent further scar expansion in the lung.

**Claims**

1. Mesenchymal stem cells for use in treating a disease selected from the group consisting of chronic obstructive pulmonary disease (COPD) and acute respiratory distress syndrome (ARDS) in an animal.

2. The mesenchymal stem cells for use according to claim 1, wherein the mesenchymal stem cells are administered in an amount effective to improve forced expiratory volume in said animal.

3. The mesenchymal stem cells for use according to claim 2, wherein the mesenchymal stem cells are administered in an amount effective to improve forced expiratory volume by at least 10% in said animal.

4. The mesenchymal stem cells for use according to any one of claims 1 to 3, wherein the mesenchymal stem cells are administered in an amount from about $1 \times 10^5$ cells per kilogram of body weight to about $1 \times 10^7$ cells per kilogram of body weight.

5. The mesenchymal stem cells for use according to claim 4, wherein the mesenchymal stem cells are administered in an amount from about $1 \times 10^6$ cells per kilogram of body weight to about $5 \times 10^6$ cells per kilogram of body weight.

6. The mesenchymal stem cells for use according to any one of claims 1 to 3, wherein the mesenchymal stem cells are administered in an amount selected from the group consisting of $0.5 \times 10^6$ cells per kilogram of body weight, $1.6 \times 10^6$ cells per kilogram of body weight, and $5 \times 10^6$ cells per kilogram of body weight.

7. Use of mesenchymal stem cells for the preparation of a pharmaceutical composition for treating a disease selected from the group consisting of chronic obstructive pulmonary disease (COPD) and acute respiratory distress syndrome (ARDS) in an animal.

8. Use according to claim 7, wherein the mesenchymal stem cells are administered in an amount sufficient to improve forced expiratory volume in said animal.

9. Use according to claim 8, wherein the mesenchymal stem cells are administered in an amount effective to improve forced expiratory volume by at least 10% in said animal.


**Patentansprüche**

1. Mesenchymale Stammzellen zur Verwendung bei der Behandlung einer Krankheit, die ausgewählt ist aus der Gruppe, bestehend aus chronisch obstruktiver Lungenerkrankung (COPD) und akutem Atemnotsyndrom (ARDS), bei einem Tier.

**2.** Mesenchymale Stammzellen zur Verwendung gemäß Anspruch 1, wobei die mesenchymalen Stammzellen in einer Menge verabreicht werden, die wirksam ist, um das forcierte exspiratorische Volumen in dem Tier zu verbessern.

**3.** Mesenchymale Stammzellen zur Verwendung gemäß Anspruch 2, wobei die mesenchymalen Stammzellen in einer Menge verabreicht werden, die wirksam ist, um das forcierte exspiratorische Volumen in dem Tier um mindestens 10% zu verbessern.

**4.** Mesenchymale Stammzellen zur Verwendung gemäß einem der Ansprüche 1 bis 3, wobei die mesenchymalen Stammzellen in einer Menge von etwa $1 \times 10^5$ Zellen pro Kilogramm Körpergewicht bis etwa $1 \times 10^7$ Zellen pro Kilogramm Körpergewicht verabreicht werden.

**5.** Mesenchymale Stammzellen zur Verwendung gemäß Anspruch 4, wobei die mesenchymalen Stammzellen in einer Menge von etwa $1 \times 10^6$ Zellen pro Kilogramm Körpergewicht bis etwa $5 \times 10^6$ Zellen pro Kilogramm Körpergewicht verabreicht werden.

**6.** Mesenchymale Stammzellen zur Verwendung gemäß einem der Ansprüche 1 bis 3, wobei die mesenchymalen Stammzellen in einer Menge verabreicht werden, die ausgewählt ist aus der Gruppe, bestehend aus $0,5 \times 10^6$ Zellen pro Kilogramm Körpergewicht, $1,6 \times 10^6$ Zellen pro Kilogramm Körpergewicht und $5 \times 10^6$ Zellen pro Kilogramm Körpergewicht.

**7.** Verwendung mesenchymaler Stammzellen zur Herstellung einer pharmazeutischen Zusammensetzung zur Behandlung einer Krankheit, die ausgewählt ist aus der Gruppe, bestehend aus chronisch obstruktiver Lungenerkrankung (COPD) und akutem Atemnotsyndrom (ARDS), bei einem Tier.

**8.** Verwendung gemäß Anspruch 7, wobei die mesenchymalen Stammzellen in einer Menge verabreicht werden, die ausreichend ist, um das forcierte exspiratorische Volumen in dem Tier zu verbessern.

**9.** Verwendung gemäß Anspruch 8, wobei die mesenchymalen Stammzellen in einer Menge verabreicht werden, die wirksam ist, um das forcierte exspiratorische Volumen in dem Tier um mindestens 10% zu verbessern.


**Revendications**

**1.** Cellules souches mésenchymateuses destinées à être utilisées dans le traitement d'une maladie sélectionnée dans le groupe consistant en la bronchopneumopathie chronique obstructive (BPCO) et le syndrome de détresse respiratoire aiguë (SDRA) chez un animal.

**2.** Cellules souches mésenchymateuses destinées à être utilisées selon la revendication 1, où les cellules souches mésenchymateuses sont administrées en une quantité efficace pour améliorer le volume expiratoire forcé chez ledit animal.

**3.** Cellules souches mésenchymateuses destinées à être utilisées selon la revendication 2, où les cellules souches mésenchymateuses sont administrées en une quantité efficace pour améliorer le volume expiratoire forcé d'au moins 10 % chez ledit animal.

**4.** Cellules souches mésenchymateuses destinées à être utilisées selon l'une quelconque des revendications 1 à 3, où les cellules souches mésenchymateuses sont administrées en une quantité comprise entre environ $1 \times 10^5$ cellules par kilogramme de poids corporel et environ $1 \times 10^7$ cellules par kilogramme de poids corporel.

**5.** Cellules souches mésenchymateuses destinées à être utilisées selon la revendication 4, où les cellules souches mésenchymateuses sont administrées en une quantité comprise entre environ $1 \times 10^6$ cellules par kilogramme de poids corporel et environ $5 \times 10^6$ cellules par kilogramme de poids corporel.

**6.** Cellules souches mésenchymateuses destinées à être utilisées selon l'une quelconque des revendications 1 à 3, où les cellules souches mésenchymateuses sont administrées en une quantité sélectionnée dans le groupe consistant en $0,5 \times 10^6$ cellules par kilogramme de poids corporel, $1,6 \times 10^6$ cellules par kilogramme de poids corporel, et $5 \times 10^6$ cellules par kilogramme de poids corporel.

**7.** Utilisation de cellules souches mésenchymateuses pour la préparation d'une composition pharmaceutique destinée au traitement d'une maladie sélectionnée dans le groupe consistant en la bronchopneumopathie chronique obstructive (BPCO) et le syndrome de détresse respiratoire aiguë (SDRA) chez un animal.

**8.** Utilisation selon la revendication 7, dans laquelle les cellules souches mésenchymateuses sont administrées en une quantité suffisante pour améliorer le volume expiratoire forcé chez ledit animal.

**9.** Utilisation selon la revendication 8, dans laquelle les cellules souches mésenchymateuses sont administrées en une quantité efficace pour améliorer le volume expiratoire forcé d'au moins 10 % chez ledit animal.

FIG. 1A

FIG. 1B

FIG. 1C

FIG. 2A

FIG. 2B

FIG. 2C

FIG. 3A

FIG. 3B

FIG. 4A

FIG. 4B

FIG. 4C

FIG. 4D

FIG. 5

FIG. 6

FIG. 7

## Improvement in FEV1 % Predicted

*p < 0.05

*Fig. 8*

### Six Minute Walk Test

Fig. 9

## Proportion of Patients with 15 Min or Less Heart Rate Recovery

Fig. 10

**REFERENCES CITED IN THE DESCRIPTION**

**Patent documents cited in the description**

- US 5486359 A
- US 6355239 B
- US 11726676 B

**Non-patent literature cited in the description**

- **PITTENGER et al.** *Science,* 1999, vol. 284, 143
- **HAYNESWORT et al.** *Bone,* 1992, vol. 13, 69
- **PROCKOP.** *Science,* 1997, vol. 276, 71
- **WAKITANI et al.** *Muscle Nerve,* 1995, vol. 18, 1417
- **WOODBURY et al.** *J. Neurosci. Res.,* 2002, vol. 69, 908
- **SANCHEZ-RAMOS et al.** *Exp. Neurol.,* 2001, vol. 171, 109
- **TOMA et al.** *Circulation,* 2002, vol. 105, 93
- **FAKUDA.** *Artif. Organs,* 2001, vol. 25, 187
- **EAVE et al.** *Ann, N.Y. Acad. Sci.,* 2001, vol. 938, 63
- **WAGERS et al.** *Gene Therapy,* 2002, vol. 9, 606
- **DEKOK et al.** *Clin. Oral Implants Res.,* 2003, vol. 14, 481
- **WU et al.** *Transplantation,* 2003, vol. 75, 679
- **NOEL et al.** *Curr. Opin. Investig. Drugs,* 2002, vol. 3, 1000
- **BALLAS et al.** *J. Cell. Biochem. Suppl.,* 2002, vol. 38, 20
- **MACKENZIE et al.** *Blood Cells Mol. Dis.,* 2002, vol. 27
- **PEREIRA et al.** *Proc. Nat. Acad. Sci.,* 1998, vol. 95, 1142
- **SCHWARTZ et al.** *Hum. Gene Ther.,* 1999, vol. 10, 2539
- **CHOPP et al.** *Neuroreport,* 2000, vol. 11, 3001
- **WU et al.** *J. Neurosci. Res.,* 2003, vol. 72, 393
- **TOMITA et al.** *Circulation,* 1999, vol. 100, 247
- **SHAKE et al.** *Ann. Thorac. Sure.,* 2002, vol. 73, 1919
- **HORWITZ et al.** *Blood,* 2001, vol. 97, 1227
- **HOROWITZ et al.** *Proc. Nat. Acad. Sci.,* 2002, vol. 99, 8932
- **FRASSONI et al.** *Int. Society for Cell Therapy,* 2002, vol. SA006
- **KOC et al.** *J. Clin. Oncol.,* 2000, vol. 18, 307
- **LE BLANC et al.** *Exp. Hematol.,* 2003, vol. 31, 890
- **POTIAN et al.** *J. Immunol.,* 2003, vol. 171, 3426
- **MAJUMDAR et al.** *J. Biomed. Sci.,* 2003, vol. 10, 228
- **TSE et al.** *Transplantation,* 2003, vol. 75, 389
- **BARTHOLOMEW et al.** *Exp. Hematol.,* 2002, vol. 30, 42
- **DEVINE et al.** *Cancer J.,* 2001, vol. 7, 576
- **DINICOLA et al.** *Blood,* 2002, vol. 99, 3838
- **MAJUMDAR et al.** *Hematother. Stem Cell Res.,* 2000, vol. 9, 841
- **HAYNESWORTH et al.** *J. Cell. Physiol.,* 1996, vol. 166, 585
- **HEANEY et al.** *Current Med. Chem.,* 2007, vol. 14 (7), 787-796
- **CALABRESE et al.** *Respir. Res.,* 2005, vol. 6, 14
- **SHIGEMURA et al.** *Circulation,* 2005, vol. 111, 1407
- **MORINO et al.** *Chest,* 2005, vol. 128, 920
- **LEBLANC et al.** *Scand J. Immunol.,* 2003, vol. 57, 11
- **DZIONEK.** *J. Immunol.,* 2000, vol. 165, 6037
- **KINGSLEY.** *J. Immunol.,* 2002, vol. 168, 1080
- **LEBLANC et al.** *Exp. Hematol.,* 2003, vol. 31, 890
- **VASSILIOU et al.** *Cell. Immunol.,* 2003, vol. 223, 120
- **HARRIS et al.** *Trends Immunol.,* 2002, vol. 23, 144
- **DENICOLA et al.** *Blood,* 2002, vol. 99, 3838
- **LEBLANC et al.** *Scand. J. Immunol.,* 2003, vol. 57, 11
- **LEBLAN et al.** *Exp. Hematol.,* 2003, vol. 31, 890
- **MILLER et al.** *Eur. Respir. J.,* 2005, vol. 26, 319-338
- **CRAPO et al.** *Am. Rev. Respir. Dis.,* 1981, vol. 123, 659-664
- *Am. J. Respir. Crit. Care Med.,* 2002, vol. 166, 111


SEQUENCE LISTING

    <110> Mesoblast International Sàrl

    <120> MESENCHYMAL STEM CELLS AND USES THEREFOR

    <130> 418-51 PCTEPT2

<140>
<141> 2008-03-21

<150> US 11/726,676
<151> 2007-03-22

<160> 4

<170> PatentIn version 3.5

<210> 1
<211> 22
<212> DNA
<213> Artificial Sequence

<220>
<223> synthetic sequences and are primers for PCR derived from human COX-1

<220>
<221> misc_feature
<222> (1)..(22)
<223> COX-1 forward primer

<400> 1
ccggatgcca gtcaggatga tg        22

<210> 2
<211> 22
<212> DNA
<213> Artificial Sequence

<220>
<223> synthetic sequences and are primers for PCR derived from human COX-1

<220>
<221> misc_feature
<222> (1)..(22)
<223> COX-1 reverse primer

<400> 2
ctagacagcc agatgctgac ag        22

<210> 3
<211> 20
<212> DNA
<213> Artificial Sequence

<220>
<223> synthetic sequences and are primers for PCR derived from human COX-2

<220>
<221> misc_feature
<222> (1)..(20)
<223> COX-2 forward primer

<400> 3
atctaccctc ctcaagtccc        20


<210> 4
<211> 21
<212> DNA
<213> Artificial Sequence


<220>
<223> primer for PCR derived from human COX-2


<220>
<221> misc_feature
<222> (1)..(21)
<223> COX-2 reverse primer


<400> 4
taccagaagg gcaggataca g        21